Europäisches Patentamt

European Patent Office

Office européen des brevets

(19)

(11) Numéro de publication : **0 092 456**
**B1**

(12) **FASCICULE DE BREVET EUROPÉEN**

(45) Date de publication du fascicule du brevet :
17.08.88

(21) Numéro de dépôt : 83400689.2

(22) Date de dépôt : 01.04.83

(51) Int. Cl.⁴ : **C 12 N 15/00, C 12 P 21/02,
A 61 K 39/12, C 07 K 7/00,
C 07 K 17/00, G 01 N 33/53**

(54) **Fragments d'ADN codant pour des polypeptides contenant au moins un déterminant antigénique des papillomavirus, notamment du type HPV 1a et polypeptides correspondants.**

(30) Priorité : 05.04.82 FR 8205887

(43) Date de publication de la demande :
26.10.83 Bulletin 83/43

(45) Mention de la délivrance du brevet :
17.08.88 Bulletin 88/33

(84) Etats contractants désignés :
IT

(56) Documents cités :
GB-A- 2 091 268
THE EMBO JOURNAL, vol. 1, no. 2, 1982, pages 231-236 O. DANOS et al.: "Human papillomavirus Ia complete DNA sequence: a novel type of genome organization among Papovaviridae"
JOURNAL OF VIROLOGY, vol. 36, no. 2, novembre 1980, pages 395-407 C.A. HEILMAN et al.: "Cloning of human papilloma virus genomic DNAs and analysis of homologous polynucleotide sequences"
VIROLOGY, vol. 118, 1982, pages 254-259 A. CLAD et al.: "Molecular cloning and partial nucleotide sequence of human Papillomavirus type Ia DNA"
JOURNAL OF VIROLOGY, vol. 40, no. 3, décembre 1981, pages 932-935 E.-M. DE VILLIERS et al.: "Molecular cloning of viral DNA from human genital warts"
EUR. J. BIOCHEM., vol. 109, 1980, pages 457-461 O. DANOS et al.: "Molecular cloning, refined physical map and heterogeneity of methylation sites of Papilloma virus type 1a DNA"
CHEMICAL ABSTRACTS, vol. 94, no. 15, 13 avril 1981, page 236, no. 116349r, Columbus, Ohio, USA W. MEINKE et al.: "Isolation and characterization of the major capsid protein of bovine papilloma virus type 1."

(73) Titulaire : **INSTITUT PASTEUR
28, rue du Docteur Roux
F-75715 Paris Cedex 15 (FR)**

(72) Inventeur : **Danos, Olivier
1, Place Rollet
F-75015 Paris (FR)**
Inventeur : **Katinka, Michael
6bis, avenue Foch
F-94160 Saint-Mandé (FR)**
Inventeur : **Yaniv, Moshe
159, rue Blomet
F-75015 Paris (FR)**

(74) Mandataire : Gutmann, Ernest et al
S.C. Ernest Gutmann - Yves Plasseraud 67, boulevard Haussmann
F-75008 Paris (FR)

EP 0 092 456 B1

CHEMICAL ABSTRACTS, vol. 94, no. 15, 13 avril 1981, page 357, no. 117603f, Columbus, Ohio, USA A.J. FARAS et al.: "Genetic variation among papillomaviruses"

CHEMICAL ABSTRACTS, vol. 90, no. 17, 23 avril 1979, page 250, no. 134848z, Columbus, Ohio, USA H. PFISTER et al.: "Characterization of proteins of human papilloma viruses (HPV) and antibody response to HPV 1"

## Description

L'invention concerne des fragments d'ADN codant pour des polypeptides contenant au moins un déterminant antigénique des papillomavirus, notamment du type HPV 1a. Elle est également relative aux produits de transformation de tels polypeptides, tels que ceux résultant de la conjugaison par l'intermédiaire de liaisons covalentes de ces polypeptides à des macromolécules support et présentant des propriétés immunogènes permettant leur utilisation, notamment soit comme moyen de diagnostic de la présence ou non de papillomavirus dans des échantillons biologiques, soit comme principe actif de vaccins susceptibles d'immuniser un hôte contre ces papillomavirus.

On sait que les papillomavirus sont susceptibles d'infecter un grand nombre d'espèces vivantes, parmi lesquelles l'homme. Ils sont responsables de la production de tumeurs bénignes, notamment de verrues au niveau de l'épithélium qu'ils colonisent. Ces tumeurs qui présentent le plus souvent un caractère régressif peuvent néanmoins dans un certain nombre de cas donner lieu à une transformation maligne. D'ailleurs les papillomavirus ont sur le plan morphologique été considérés comme apparentés à des virus de polyome, tels que les virus connus sous les désignations SV 40, BKV etc. Ces différents types de virus ont en effet en commun une structure de capside icosahédrique renfermant une double hélice d'ADN associée avec des histones. Malgré l'impossibilité reconnue de cultiver ces papillomavirus sur des cellules épithéliales ou autres cellules, au sein d'une culture de tissu, O. DANOS et al ont récemment réussi à cloner le génome entier du virus de papillome humain, du type 1a, dans une souche d'Escherichia coli (Eur. J. Biochem., 109, 457-461 (1980)).

La présente invention résulte de la découverte de certaines des séquences du génome de ce papillomavirus, qui sont susceptibles de coder pour des peptides ou polypeptides susceptibles de contenir des déterminants antigéniques permettant d'envisager leur utilisation comme principe actif de vaccins, le cas échéant après couplage avec des macromolécules support, du moins pour les plus petits d'entre eux.

Il est à cet égard significatif que ces séquences sont tout à fait distinctes de toutes séquences contenues dans les génomes de virus de polyomes mentionnés plus haut. Ces séquences se révèlent en fait être portées par un seul des brins du génome du papillomavirus, comme en témoignent les analyses de séquences du génome entier du papillomavirus.

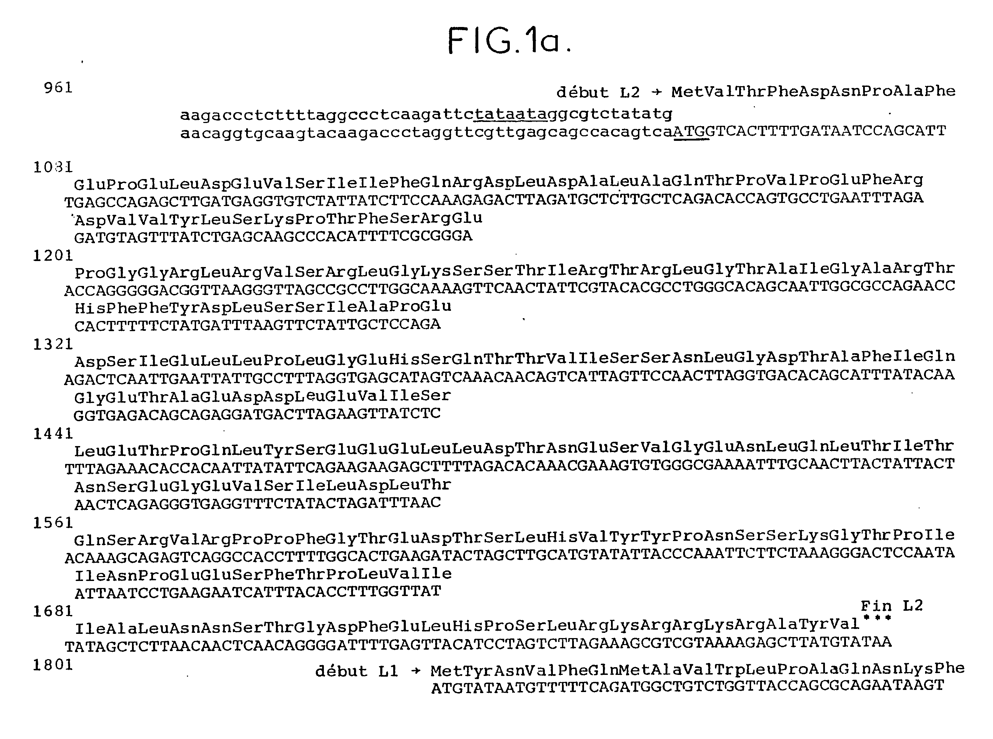

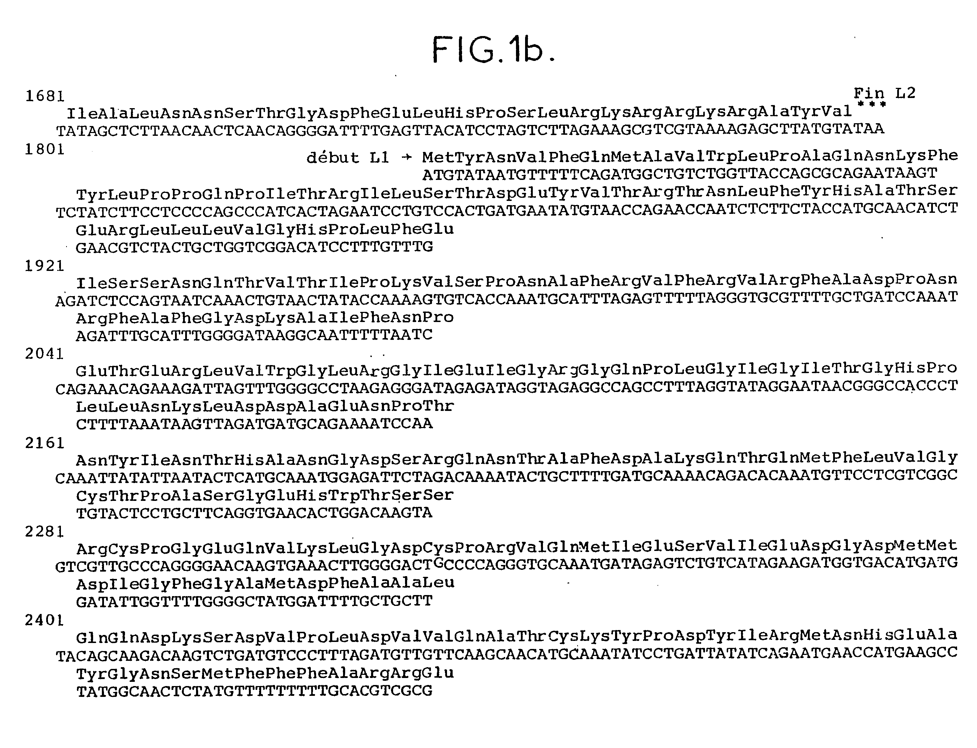

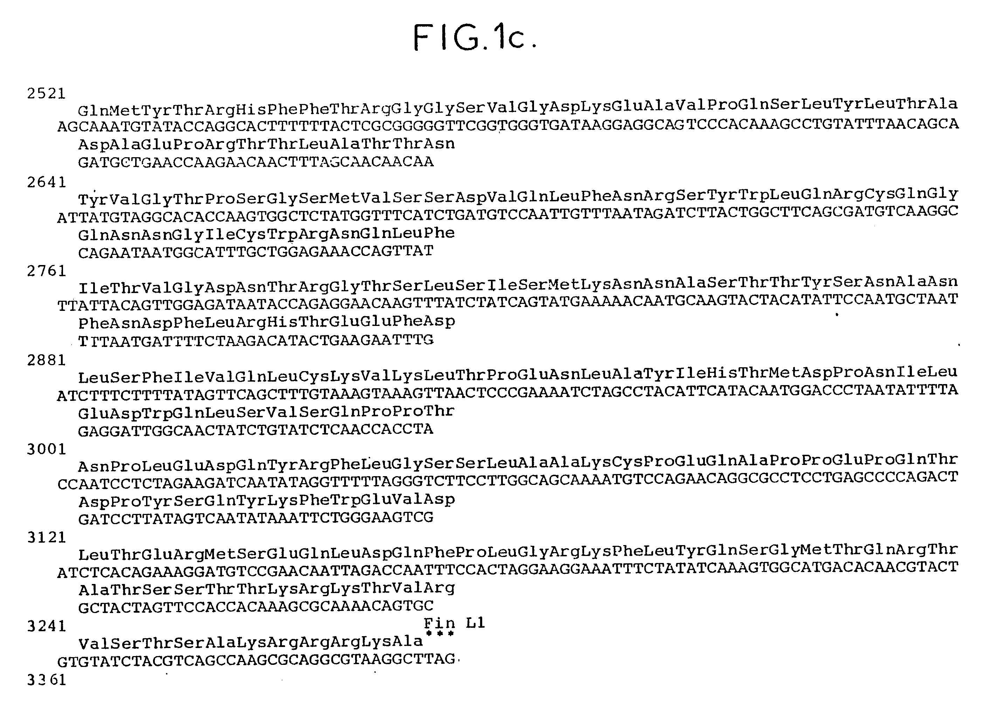

Le fragment d'ADN selon l'invention est caractérisé en ce qu'il est constitué par une séquence de nucléotides capable de coder pour une ou des protéines de structure d'un papillomavirus humain, notamment du type la (HPV 1a) ou pour un ou des polypeptides ayant en commun avec ces protéines une séquence contenant au moins un déterminant antigénique caractéristique de ces papillomavirus, cette séquence de nucléotides étant contenue soit dans la région L1 montrée dans les figures 1b et 1c soit dans la région L2 montrée dans la fig. 1a, soit encore pour partie dans la région L1 et pour partie dans la région L2 ladite séquence étant le cas échéant complétée par des fragments d'ADN issus du génome du papillomavirus et normalement associés dans celui-ci audit génome, et comportant au plus une centaine de nucléotides.

Pour la commodité de l'exposé, il sera ci-après fait référence aux dessins dans lesquels :
- les fig. 1a, 1b et 1c sont représentatives de la structure d'une partie de l'un des brins du génome de HPV 1a, plus particulièrement de celui dont la séquence se lit de l'extrémité 5' à l'extrémité 3' correspondante ;
- les fig. 2a et 2b sont des représentations schématiques des parties qui, dans les brins respectifs du génome du HPV 1, sont susceptibles d'être exprimées sous forme de polypeptide et la fig. 2c est une représentation schématique du génome d'un papillomavirus bovin BPV 1 ;
- la fig. 3 est représentative des structures comparées d'un fragment d'ADN préféré, conforme à l'invention, issu de HPV 1a et d'un fragment correspondant du génome de papillomavirus bovin du type BPV 1.

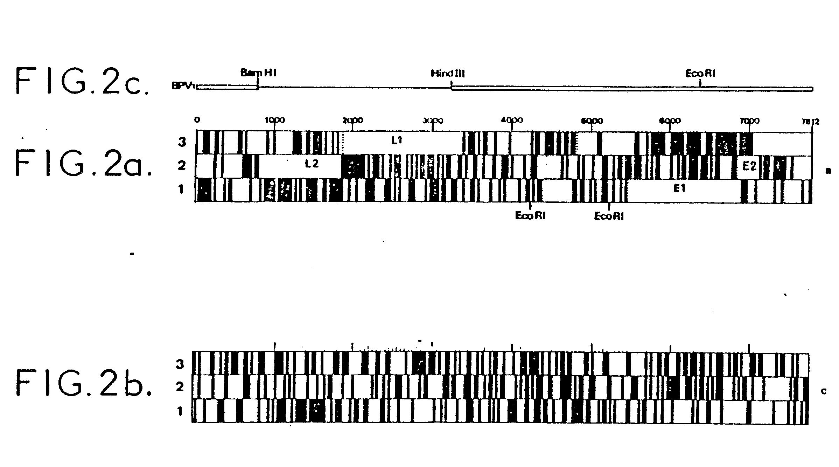

Les figures 1a, d'une part, et 1b, 1c, d'autre part, font apparaître les structures des régions L1 et L2 de celui des brins du génome du papillomavirus HPV 1a qui le porte. Ces figures font également apparaître les résidus aminoacyle codés par les triplets successifs que définissent les nucléotides du brin en question, ces protéines correspondant à des phases de lecture distinctes des séquences de nucléotides correspondantes. C'est notamment ce que fait apparaître clairement la fig. 1a, plus particulièrement au niveau des nucléotides numérotés 1870 à 1881, à compter de l'extrémité 5' (non représentée dans la fig. 1). Les positions relatives des régions L1 et L2 sur le brin correspondant, dans la direction de lecture s'étendant de l'extrémité 5' à l'extrémité opposée 3' de ce même brin, résultent de l'examen de la fig. 2a, laquelle fait apparaître les distributions des régions susceptibles de donner lieu à l'expression, notamment lorsque les fragments d'ADN correspondants, préalablement insérés dans un vecteur, sont utilisés pour transformer des micro-organismes appropriés.

La fig. 2a correspond aux trois phases de lecture possible du brin correspondant, dont l'extrémité 5' serait située à la gauche et l'extrémité 3' à la droite de la fig. 2a. Les codons sont examinés par groupes de dix, chaque barre noire correspondant à ceux desdits groupes qui, dans la phase de lecture considérée ; comportent un codon d'arrêt. Les barres verticales en pointillé correspondent au premier codon ATG présent dans chacune des séquences qui s'ensuivent et qui sont dépourvues de codons d'arrêt. Les deux sites EcoRl aux positions 4237 et 5240 visent à faciliter l'orientation schématique des séquences résultant des trois phases de lecture possibles, repérées dans la gauche de la figure par les chiffres 3, 2 et 1. La fig. 2a rend compte des positions relatives des régions L1 et L2 dont il a été question plus haut.

La fig. 2b rend compte des possibilités de lecture dans les mêmes conditions du brin complémentaire de l'ADN du génome de HPV 1a. On constate la présence d'un nombre considérable de codons d'arrêt s'étendant sur la quasitotalité du brin correspondant, quelle que soit la phase de lecture envisagée.

Comme il a été indiqué plus haut, l'invention concerne entre autres des fragments d'ADN susceptibles de contenir une zone commune à la région L1 et à la région L2, une telle situation pouvant se présenter à l'occasion de l'épissure qui peut se produire entre les deux régions, à l'occasion des opérations de transcription de ces régions, au sein même des cellules transformées.

Cependant, de préférence, l'invention concerne des fragments d'ADN ayant des séquences communes de nucléotides avec la susdite région L1 de HPV 1a.

La localisation plus spécifique de celles des séquences contenues dans les régions L1 et L2 sus-indiquées, qui sont susceptibles de porter un déterminant antigénique caractéristique des papillomavirus, plus particulièrement de HPV 1a, peut s'opérer de toute façon en soi connue, notamment comme suite à la fragmentation des séquences d'ADN correspondantes, que ce soit par des enzymes de restriction appropriées ou par clivage chimique, par intégration des fragments obtenus dans un vecteur et transformation d'un micro-organisme approprié à l'aide des vecteurs obtenus et permettant l'expression, par séparation des peptides obtenus, ceux-ci étant ensuite, le cas échéant après couplage avec une macromolécule support, utilisés pour induire la production d'anticorps dans un hôte vivant. Sont alors retenus au titre des fragments selon l'invention ceux qui sont susceptibles de produire des anticorps capables de neutraliser le HPV 1a dans son entier.

Une séquence de nucléotides préférée selon l'invention consiste en celle qui code pour la séquence peptidique de formule :
Leu-Asp-Gln-Phe-Pro-Leu-Gly-Arg-Lys-Phe-Leu.

Le peptide limité à cette séquence peptidique est en particulier codé par la séquence de nucléotides :
TTA GAC CAA TTT CCA CTA GGA AGG AAA TTT CTA.

Cette séquence de nucléotides correspond à ceux qui s'étendent des positions 3148 à 3180 dans la fig. 1c.

Un autre fragment préféré selon l'invention contient une séquence codant pour la séquence peptidique suivante :
Ala-Lys-Arg-Arg-Arg-Lys.

Le peptide correspondant à cette séquence peptidique est en particulier codé par la séquence de nucléotides :
GCC AAG CGC AGG CGT AAG.

L'invention concerne naturellement également tous fragments d'ADN codant pour des protéines de structure d'autres papillomavirus, tels que les papillomavirus CRPV (abréviation de « cottontail rabbit papillomavirus ») et BPV1 (abréviation de « bovine papillomavirus du type 1 »). Il en est encore également de même des peptides codés par ces fragments d'ADN. Parmi ces peptides figurent notamment ceux correspondant à des séquences d'ADN portées par les virus CRPV et BPV1 susmentionnés, ces peptides étant caractérisés par la séquence suivante :
CPRV : Leu-Asp-Gln-Tyr-Pro-Leu-Gly-Arg-Lys-Phe-Leu
BPV1 : Leu-Asp-Gln-Phe-Pro-Leu-Gly-Arg-Arg-Phe-Leu.

Font également partie de l'invention les séquences d'ADN dont les triplets se distinguent de ceux qui ont été évoqués ci-dessus par une structure nucléotidique différente, dans la mesure cependant où ils codent soit pour des acides aminés identiques ou encore des acides aminés « équivalents », étant entendu que l'expression « équivalents » vise ici à désigner tout acide aminé susceptible d'être substitué à l'un des acides aminés de la structure de base sans cependant modifier essentiellement les propriétés immunogéniques des peptides correspondants. En d'autres termes, les acides aminés équivalents seront ceux qui permettent l'obtention d'une séquence peptidique modifiée, qui le cas échéant après couplage avec un support macromoléculaire adéquat, permet l'induction in vivo d'anticorps qui restent capables de neutraliser soit le peptide de base, soit encore plus généralement le papillomavirus correspondant HPV 1a.

Ces aminoacyles équivalents peuvent être déterminés soit en s'appuyant sur leur homologie de structure, soit sur les résultats des essais d'immunogénicités croisées auxquels les différentes séquences de peptides obtenus sont susceptibles de donner lieu.

A titre d'exemple, on mentionnera les possibilités de substitutions susceptibles d'être souvent effectuées, sans qu'il en résulte une modification approfondie de l'immunogénicité des peptides modifiés correspondants, les remplacements, par exemple de la leucine par la valine ou l'isoleucine, de l'acide aspartique par l'acide glutamique, de la glutamine par l'asparagine, de l'arginine par la lysine, etc., les substitutions inverses étant naturellement envisageables dans les mêmes conditions.

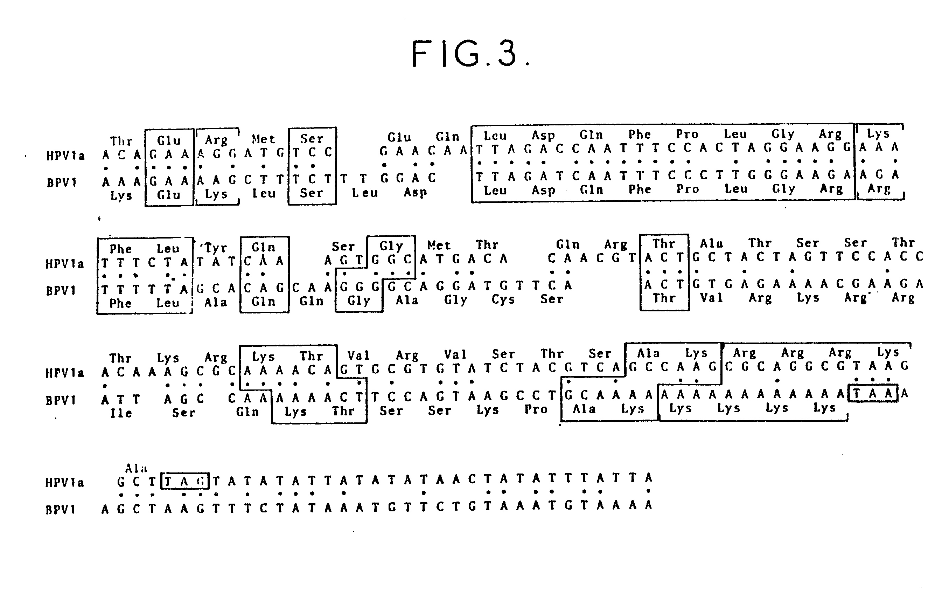

A cet égard, il est intéressant de relever la présence dans le génome de papillomavirus bovin du type BPV 1, de séquences présentant un certain niveau d'homologie avec des séquences correspondantes de HPV 1a, comme il résulte notamment de l'examen de la fig. 3.

La fig. 3 fait en effet apparaître des homologies qui peuvent être relevées entre la zone d'extrémité 3' (s'étendant du nucléotide numéroté 3246 à partir de l'extrémité 5' du génome de HPV 1a jusqu'au nucléotide 3476) de la région L1 selon le code de lecture correspondant, et par ailleurs une région correspondante du génome de BPV 1. Cette dernière région a été définie par analyse séquentielle d'un recombinant obtenu entre le vecteur M13 décrit par ROTHSTEIN et WU (Gen. 15, 1981, 167-176) et/ou MESSING J. et al (Nucl. Acids Res. (1981) 9, 309321) et un fragment de BPV 1 délimité par des extrémités Bgl II débutant environ 10 nucléotides avant l'extrémité du site Hind III de BPV 1 (schématiquement représenté à la fig. 2c). Les points placés entre les lettres en regard d'une séquence nucléotidique représentée visent à souligner le caractère identique des nucléotides en question, les intervalles blancs laissés libres dans chacune des séquences n'ayant d'autre fin que celle de faire apparaître de façon plus nette encore les homologies existantes. Les séquences peptidiques communes codées par les séquences correspondantes contenues à l'intérieur des fragments considérés à la fig. 3 apparaissent dans les encadrés représentés.

Font donc également partie des fragments d'ADN particuliers de l'invention, ceux qui codent pour les peptides :

Leu-Asp-Gln-Phe-Pro-Leu-Gly-Arg-Arg-Phe-Leu
Ala-Lys-Lys-Lys-Lys-Lys.

Au titre des fragments nucléotidiques particuliers entrant dans le cadre de l'invention, on mentionnera naturellement également ceux qui sont contenus dans le fragment susmentionné de BPV 1, plus particulièrement :

TTA GAT GAA TTT CCC TTG GGA AGA AGA
TTT TTA, GCA AAA AAA AAA AAA AAA.

L'invention concerne naturellement également tous fragments d'ADN équivalents, dans les conditions telles qu'elles ont été définies plus haut.

Les différentes séquences d'ADN telles qu'elles ont été décrites ci-dessus, peuvent être obtenues, comme il a déjà été indiqué, notamment par fragmentation du génome et récupération des fragments appropriés correspondants, contenant les enchaînements de nucléotides correspondant aux séquences contenues dans les susdites régions L1 et L2 ou encore à des régions plus petites contenant néanmoins des déterminants antigéniques spécifiques à l'égard du virus entier. En ce qui concerne les fragments les plus petits, notamment ceux qui codent pour un nombre limité d'acides aminés, tels qu'ils ont été illustrés par les exemples, on peut également avoir recours à la synthèse chimique des nucléotides correspondants, selon des méthodes à ce jour bien connues, les séquences obtenues pouvant alors être utilisées comme insérats susceptibles d'être incorporés dans un vecteur permettant la transformation des micro-organismes appropriés à leur expression.

En ce qui concerne les peptides eux-mêmes, on peut également avoir recours, surtout lorsqu'il s'agit de peptides ne comprenant qu'un nombre limité de résidus aminoacyles, à des techniques elles-mêmes connues de synthèse chimique.

A cet égard, on pourra avoir recours au mode de synthèse en solution homogène décrit par HOUBENWEYL dans l'ouvrage intitulé « Methodem der Organischen Chemie » (Méthode de la chimie organique) édité par E. Wünsch., vol. 15-I et II, THIEME, Stuttgart 1974.

Cette méthode de synthèse consiste à condenser successivement deux à deux les aminoacyles successifs dans l'ordre requis, ou à condenser des aminoacyles et des fragments préalablement formés et contenant déjà plusieurs résidus aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments à l'exception des fonctions amine de l'un et carboxyle de l'autre ou vice versa, qui doivent normalement intervenir dans la formation des liaisons peptidiques, notamment après activation de la fonction carboxyle, selon les méthodes bien connues dans la synthèse des protéines. En variante, on pourra avoir recours à des réactions de couplage mettant en jeu des réactifs de couplage classique, du type carbodiimide, tels que par exemple 1a 1-éthyl-3-(3-diméthylaminopropyl)-carbodiimide. Lorsque l'aminoacyle mis en œuvre possède une fonction amine supplémentaire (cas de la lysine par exemple) ou une autre fonction acide (cas par exemple de l'acide glutamique), ces fonctions seront par exemple protégées, par des groupes carbobenzoxy ou t-butyloxycarbonyle, en ce qui concerne les fonctions amine, ou par des groupes t-butylester, en ce qui concerne les fonctions carboxyliques. Il en ira de même de la protection de toute autre fonction réactive. Par exemple lorsque l'un des aminoacyles considérés contient une fonction SH (par exemple le cystéine), on pourra avoir recours à un groupe acétamidométhyle ou formamidométhyle.

Dans le cas de la synthèse progressive, acide aminé par acide aminé, la synthèse débute de préférence par la condensation de l'amino acide C-terminal avec l'aminoacide qui correspond à l'aminoacyle voisin dans la séquence désirée et ainsi de suite, de proche en proche, jusqu'à l'acide aminé N-terminal. Selon une autre technique préférée de l'invention, on a recours à celle décrite par R. D. MERRIFIELD dans l'article intitulé « Solid phase peptide synthesis » (J. Am. Chem. Soc., 45, 2149-2154).

Pour fabriquer une chaîne peptidique selon le procédé de MEERIFIELD, on a recours à une résine polymère très poreuse, sur laquelle on fixe le premier aminoacide C-terminal de la chaîne. Cet aminoacide est fixé sur la résine par l'intermédiaire de son groupe carboxylique et sa fonction amine est protégée, par exemple par le groupe t-butyloxycarbonyle.

Lorsque le premier aminoacide C-terminal est ainsi fixé sur la résine, on enlève le groupe protecteur de la fonction amine en lavant la résine avec un acide.

Dans le cas où le groupe protecteur de la fonction amine est le groupe t-butyloxycarbonyle, il peut être éliminé par lavage de la résine à l'aide

d'acide trifluoroacétique.

On fait ensuite réagir le deuxième aminoacide qui fournit le second aminoacyle de la séquence recherchée, à partir du résidu aminoacyle C-terminal sur la fonction amine déprotégée du premier aminoacide C-terminal fixé sur la chaîne. De préférence, la fonction carboxyle de ce deuxième aminoacide est activée, par exemple par la dicyclohexylcarbodiimide, et la fonction amine est protégée, par exemple par le t-butyloxycarbonyle.

On obtient ainsi la première partie de la chaîne peptidique recherchée, qui comporte deux aminoacides, et dont la fonction amine terminale est protégée. Comme précédemment, on déprotège la fonction amine et on peut ensuite procéder à la fixation du troisième aminoacyle, dans les conditions analogues à celles de l'addition du deuxième aminoacide C-terminal.

On fixe ainsi, les uns après les autres, les acides aminés, qui vont constituer la chaîne peptidique sur le groupe amine chaque fois déprotégé au préalable de la portion de la chaîne peptidique déjà formée, et qui est rattachée à la résine.

Lorsque la totalité de la chaîne peptidique désirée est formée, on élimine les groupes protecteurs des différents aminoacides constituant la chaîne peptidique et on détache le peptide de la résine par exemple à l'aide d'acide fluorhydrique.

L'invention concerne également les produits obtenus par la conjugaison (par liaison covalente) entre les peptides, tels qu'ils ont été définis plus haut, et un support macromoléculaire du genre de ceux qui peuvent être utilisés pour la constitution de principes actifs immunogènes.

De tels supports sont bien connus des spécialistes. Il peut s'agir de supports naturels, tels que des sérums albumines, de préférence humaines lorsqu'il s'agit de vaccins destinés à l'homme, ou animales, lorsqu'il s'agit de vaccins destinés à l'usage vétérinaire. On mentionnera encore à titre d'exemple de supports macromoléculaires naturels les ovalbumines, la toxine tétanique, etc. ayant des poids moléculaires de préférence supérieurs à 20.000.

On peut également avoir recours à des supports macromoléculaires synthétiques tels que des polypeptides synthétiques. A titre d'exemple, on mentionne des polylysines, portant le cas échéant des chaînes latérales de polyalanine (les motifs alanyles étant dextrogyres et/ou lévogyres). On peut encore avoir recours aux chaînes synthétiques, telles que celles décrites par exemple dans la demande de brevet français n° 79 00819.

Les principes actifs selon l'invention sont utilisables notamment pour protéger des sujets contre les papillomavirus, par exemple préalablement à la réalisation d'un traitement immunosuppresseur.

Une importante application réside dans la production de sérums ou d'anticorps davantage purifiés, en vue de réaliser des réactifs de diagnostic de papillomavirus qui peuvent être utilisés dans des tests de routine, notamment à l'occasion d'examens de frottis vaginaux, ou autres examens gynécologiques, par exemple dans le cas de dépistage de certains types de cancer du col utérin. Ils peuvent encore servir à des tests de diagnostic préventif en dermatologie.

L'invention concerne naturellement aussi toutes les compositions de vaccins, dans lesquelles les susdits principes actifs sont associés à des véhicules pharmaceutiques permettant leur administration par voie parentérale, orale ou autre. Ces compositions contiennent éventuellement également un adjuvant immunologique du type muramyl-peptide, permettant le renforcement de la réaction immunitaire à l'égard du principe vaccinant.

Ces compositions peuvent être utilisées en médecine humaine ou vétérinaire, les séquences immunogènes de papillomavirus mises en œuvre étant alors, le cas échéant originaires, notamment en ce qui concerne celles qui comportent un nombre de résidus aminoacyle élevé, des papillomavirus colonisant préférentiellement les tissus des espèces auxquelles le vaccin est destiné.

D'une façon générale, l'invention concerne naturellement tout fragment d'ADN dont le produit d'expression dans un micro-organisme approprié contient au moins l'un des déterminants antigéniques d'un papillomavirus, humain ou animal, ce fragment étant caractérisé en ce qu'il comporte une séquence de nucléotides, elle-même contenue ou analogue à celle contenue soit dans la région L1, soit dans la région L2, soit encore pour partie dans la région L1 et pour partie dans la région L2 de celui des brins du génome de papillomavirus qui les comporte et qui sont capables de coder pour des protéines de structure du virus ou pour des polypeptides ayant en commun avec ces protéines une séquence contenant au moins un déterminant antigénique caractéristique des papillomavirus.

Il est entendu que les revendications qui suivent couvrent non seulement les fragments d'ADN et les fragments de peptides correspondants, mais également tous les équivalents susceptibles d'être réalisés, notamment mais non exclusivement en conformité avec les indications qui ont été formulées dans la présente description.

L'invention concerne également toutes séquences d'ADN extraites du génome des papillomavirus, autres que celles qui ont été évoquées ci-dessus, notamment les séquences E1 (nucléotides 5473-6919) et E2 (nucléotides 6863-16) (sur la fig. 2a au niveau des phases de lecture 1 et 2). Les peptides exprimés par ces séquences peuvent induire in vivo la formation d'anticorps utilisables pour détecter des lésions provoquées par des papillomavirus (et contenant des fragments de protéines et le DNA viral) dans des réactions classiques d'antigènes anticorps dépistables par les réactions classiques d'immunofluorescence ou d'immunoenzymatiques, etc.

**Revendications**

1. Fragment d'ADN, caractérisé en ce qu'il est constitué par une séquence de nucléotides capable de coder pour une ou des protéines de structure d'un papillomavirus humain, notamment du type 1a (HPV 1a) ou pour un ou des polypeptides ayant en commun avec ces protéines une séquence contenant au moins un déterminant antigénique caractéristique de ces papillomavirus, cette séquence de nucléotides étant contenue soit dans la région L1 montrée dans les figures 1b et 1c, soit dans la région L2 montrée dans la fig. 1a, soit encore pour partie dans la région L1 et pour partie dans la région L2.

2. Fragment d'ADN selon la revendication 1 comprenant au plus une centaine de nucléotides tous issus du génome du papillomavirus HPV 1a.

3. Fragment d'ADN selon la revendication 1 ou 2 caractérisé en ce qu'il contient une séquence de nucléotides codant pour le peptide :
Leu-Asp-Gln-Phe-Pro-Leu-Gly-Arg-Lys-Phe-Leu.

4. Fragment d'ADN caractérisé en ce qu'il code pour le peptide de la revendication 3.

5. Fragment d'ADN selon la revendication 4, caractérisé par la séquence de nucléotides :
TTA GAC CAA TTT CCA CTA GGA AGG AAA TTT CTA.

6. Fragment d'ADN codant pour le peptide :
Leu-Asp-Gln-Phe-Pro-Leu-Gly-Arg-Arg-Phe- Leu.

7. Fragment d'ADN selon la revendication 6, caractérisé par la séquence de nucléotides :
TTA GAT CAA TTT CCC TTG GGA AGA AGA TTT TTA.

8. Fragment d'ADN selon la revendication 1 ou 2, caractérisé en ce qu'il contient une sequence de nucléotides codant pour le peptide :
Ala-Lys-Arg-Arg-Arg-Lys.

9. Fragment d'ADN caractérisé en ce qu'il code pour le peptide de la revendication 8.

10. Fragment d'ADN codant pour le peptide :
Ala-Lys-Lys-Lys-Lys-Lys.

11. Fragment d'ADN selon la revendication 8, caractérisé en ce qu'il contient la séquence de nucléotides suivante :
GCC AAG CGC AGG CGT AAG.

12. Fragment d'ADN selon la revendication 10 dont la séquence est la suivante :
GCA AAA AAA AAA AAA AAA.

13. Peptide dont la séquence d'aminoacides est contenue dans la séquence de la protéine L1 illustrée dans les figures 1b et 1c et qui contient au moins l'une des séquences peptidiques suivantes :
a) Leu-Asp-Gln-Phe-Pro-Leu-Gly-Arg-Lys-Phe-Leu
b) Ala-Lys-Arg-Arg-Arg-Lys

14. Peptide constitué par l'un ou l'autre des peptides :
a) Leu-Asp-Gln-Phe-Pro-Leu-Gly-Arg-Lys-Phe-Leu
b) Ala-Lys-Arg-Arg-Arg-Lys.

15. Peptide constitué par l'un ou par l'autre des peptides suivants :
a) Leu-Asp-Gln-Phe-Pro-Leu-Gly-Arg-Arg-Phe-Leu
b) Ala-Lys-Lys-Lys-Lys-Lys.

16. Un peptide immunogène capable d'induire in vivo la production d'anticorps actifs à l'égard des papillomavirus, notamment de celui du type HPV 1a, caractérisé en ce qu'il contient l'un au moins des peptides codés par l'un au moins des fragments d'ADN selon l'une quelconque des revendications 1 à 12, ou l'un au moins des peptides selon l'une quelconque des revendications 13 à 15.

17. Peptide immunogène selon l'une quelconque des revendications 13 à 16, caractérisé en ce qu'il est conjugué à un support macromoléculaire.

18. Les compositions de vaccin contenant à titre de principe actif immunogène l'un des peptides tels que définis dans l'une quelconque des revendications 13 à 16 ou le peptide immunogène de la revendication 17.

19. Les anticorps tels qu'induits in vivo par les peptides selon les revendications 13 à 17.

**Claims**

1. DNA fragment characterized in that it is formed of a sequence of nucleotides capable of coding for one or several structural proteins of a human papillomavirus, particularly of the 1a type (HPV 1a) or for one or several polypeptides having in common with these proteins a sequence containing at least one antigenic determinant characteristic of these papillomaviruses, said sequence of nucleotides being contained either in the L1 region shown on Figures 1b and 1c, or in the L2 region shown on Figure 1a, or still for part of it in the L1 region and for another part of it in the L2 region.

2. DNA fragment according to claim 1 which comprises at least one hundred nucleotides all originating from the HPV la papillomavirus genome.

3. DNA fragment according to claim 1 or 2, characterized in that it contains a sequence of nucleotides coding for the peptide.
Leu-Asp-Gln-Phe-Pro-Leu-Gly-Arg-Lys-Phe-Leu.

4. DNA fragment characterized in that it codes for the peptide of claim 3.

5. DNA fragment according to claim 4, characterized by the sequence of nucleotides :
TTA GAC CAA TTT CCA CTA GGA AGG AAA TTT CTA.

6. DNA fragment coding for the peptide :
Leu-Asp-Gln-Phe-Pro-Leu-Gly-Arg-Arg-Phe-Leu.

7. DNA fragment according to claim 6, characterized by the sequence of nucleotides :
TTA GAC CAA TTT CCC TTG GGA AGA AGA TTT TTA.

8. DNA fragment according to claim 1 or 2, characterized in that it contains a sequence of nucleotides coding for the peptide :
Ala-Lys-Arg-Arg-Arg-Lys

9. DNA fragment characterized in thatit codes for the peptide of claim 8.

10. DNA fragment coding for the peptide :
Ala-Lys-Lys-Lys-Lys-Lys.

11. DNA fragment according to claim 8, characterized in that it contains the following sequence of nucleotides :
GCC AAG CGC AGG CGT AAG.

12. DNA fragment according to claim 10 with sequence is the following :

GCA AAA AAA AAA AAA AAA

13. Peptide whose aminoacid sequence is contained within the sequence of the L1 protein illustrated in Figures 1b and 1c and which contains at least one of the following peptidic sequences :

a) Leu-Asp-Gln-Phe-Pro-Leu-Gly-Arg-Lys-Phe-Leu

b) Ala-Lys-Arg-Arg-Arg-Lys

14. Peptide constituted by one or the other of the following peptides

a) Leu-Asp-Gln-Phe-Pro-Leu-Gly-Arg-Lys-Phe-Leu

b) Ala-Lys-Arg-Arg-Arg-Lys.

15. Peptide constituted by one or the other of the following peptides :

a) Leu-Asp-Gln-Phe-Pro-Leu-Gly-Arg-Arg-Phe-Leu

b) Ala-Lys-Lys-Lys-Lys-Lys.

16. An immunogenic peptide capable of inducing in vivo the production of antibodies active against said papillomaviruses, particularly that of HPV 1a type, characterized in that it contains at least one of the peptides encoded by one at least of the DNA fragments according to anyone of claims 1 to 12, or at least one of the peptides according to anyone of claims 13 to 15.

17. Immunogenic peptide according to any of claims 13 to 16, characterized in that it is conjugated to a macromolecular support.

18. Vaccin compositions which contained as an immunogenic active principle one of the peptides as defined in anyone of claims 13 to 16 or the immunogenic peptide of claim 17.

19. The antibodies such as induced in vivo by the peptides according to claims 13 to 17.

**Patentansprüche**

1. DNS-Fragment, dadurch gekennzeichnet, daß es aus einer Nucleotidsequenz besteht, die für mindestens ein Strukturprotein des Papillomavirus des Menschen zu codieren befähigt ist, insbesondere des Typs 1a (HPV 1a), oder für mindestens ein Polypeptid, welches mit diesen Proteinen eine Sequenz gemeinsam hat, die mindestens eine für das Papillomavirus charakteristische antigene Determinante enthält, wobei die Nucleotidsequenz entweder in dem in den Figuren 1b und 1c dargestellten Bereich L1, oder in dem in Figur 1a dargestellten Bereich L2 oder auch teilweise im Bereich L1 und teilweise im Bereich L2 enthalten ist.

2. DNS-Fragment nach Anspruch 1, das zusätzlich etwa 100 Nucleotide enthält, die alle vom Genom des Papillomavirus HPV 1a stammen.

3. DNS-Fragment nach Anspruch 1 oder 2, dadurch gekennzeichnet, daß es eine Nucleotidsequenz enthält, die für das Peptid :

Leu-Asp-Gln-Phe-Pro-Leu-Gly-Arg-Lys-Phe-Leu codiert.

4. DNS-Fragment, dadurch gekennzeichnet, daß es für das Peptid nach Anspruch 3 codiert.

5. DNS-Fragment nach Anspruch 4, gekennzeichnet durch die Nucleotidsequenz :

TTA GAC CAA TTT CCA CTA GGA AGG AAA TTT CTA.

6. DNS-Fragment, das für das Peptid :

Leu-Asp-Gln-Phe-Pro-Leu-Gly-Arg-Arg-Phe-Leu codiert.

7. DNS-Fragment nach Anspruch 6, gekennzeichnet durch die Nucleotidsequenz :

TTA GAT CAA TTT CCC TTG GGA AGA AGA TTT TTA.

8. DNS-Fragment nach Anspruch 1 oder 2, dadurch gekennzeichnet, daß es eine Nucleotidsequenz enthält, die für das Peptid :

Ala-Lys-Arg-Arg-Arg-Lys codiert.

9. DNS-Fragment, dadurch gekennzeichnet, daß es für das Peptid nach Anspruch 8 codiert.

10. DNS-Fragment, das für das Peptid :

Ala-Lys-Lys-Lys-Lys-Lys codiert.

11. DNS-Fragment nach Anspruch 8, dadurch gekennzeichnet, daß es die folgende Nucleotidsequenz enthält :

GCC AAG CGC AGG CGT AAG

12. DNS-Fragment nach Anspruch 10 mit der folgenden Sequenz :

GCA AAA AAA AAA AAA AAA.

13. Peptid, dessen Aminosäuresequenz in der Sequenz des Proteins L1 enthalten ist, welche in den Figuren 1b und 1c dargestellt ist, und die mindestens eine der folgenden Peptidsequenzen enthält :

a) Leu-Asp-Gln-Phe-Pro-Leu-Gly-Arg-Lys-Phe-Leu

b) Ala-Lys-Arg-Arg-Arg-Lys.

14. Peptid, bestehend aus einem der Peptide :

a) Leu-Asp-Gln-Phe-Pro-Leu-Gly-Arg-Lys-Phe-Leu

b) Ala-Lys-Arg-Arg-Arg-Lys.

15. Peptid, bestehend aus einem der folgenden Peptide :

a) Leu-Asp-Gln-Phe-Pro-Leu-Gly-Arg-Arg-Phe-Leu

b) Ala-Lys-Lys-Lys-Lys-Lys

16. Immunogenes Peptid, das dazu in der Lage ist, in vivo die Erzeugung von gegen Papillomavirus, insbesondere des Typs HPV 1a, wirksamen Antikörpern anzuregen, dadurch gekennzeichnet, daß es mindestens eines der Peptide enthält, die durch mindestens eines der DNS-Fragmente nach einem der Ansprüche 1 bis 12 codiert werden, oder mindestens eines der Peptide nach einem der Ansprüche 13 bis 15.

17. Immunogenes Peptid nach einem der Ansprüche 13 bis 16, dadurch gekennzeichnet, daß es an einen makromolekularen Träger gebunden ist.

18. Impfstoffe, enthaltend als immunogenen Wirkstoff ein Peptid nach einem der Ansprüche 13 bis 16 oder ein immunogenes Peptid nach Anspruch 17.

19. In vivo durch die Peptide nach den Ansprüchen 13 bis 17 induzierte Antikörper.

# FIG.1a.

début L2 → MetValThrPheAspAsnProAlaPhe

aagaccctcttttaggccctcaagattc<u>tataata</u>ggcgtctatatg

aacaggtgcaagtacaagaccctaggttcgttgagcagccacagtca<u>ATG</u>GTCACTTTTGATAATCCAGCATT

GluProGluLeuAspGluValSerIleIlePheGlnArgAspLeuAspAlaLeuAlaGlnThrProValProGluPheArg
TGAGCCAGAGCTTGATGAGGTGTCTATTATCTTCCAAAGAGACTTAGATGCTCTTGCTCAGACACCAGTGCCTGAATTTAGA

AspValValTyrLeuSerLysProThrPheSerArgGlu
GATGTAGTTTATCTGAGCAAGCCCACATTTTCGCGGGA

ProGlyGlyArgLeuArgValSerArgLeuGlyLysSerSerThrIleArgThrArgLeuGlyThrAlaIleGlyAlaArgThr
ACCAGGGGGACGGTTAAGGGTTAGCCGCCTTGGCAAAAGTTCAACTATTCGTACACGCCTGGGCACAGCAATTGGCGCCAGAACC

HisPhePheTyrAspLeuSerSerIleAlaProGlu
CACTTTTTCTATGATTTAAGTTCTATTGCTCCAGA

AspSerIleGluLeuLeuProLeuGlyGluHisSerGlnThrThrValIleSerSerAsnLeuGlyAspThrAlaPheIleGln
AGACTCAATTGAATTATTGCCTTTAGGTGAGCATAGTCAAACAACAGTCATTAGTTCCAACTTAGGTGACACAGCATTTATACAA

GlyGluThrAlaGluAspAspLeuGluValIleSer
GGTGAGACAGCAGAGGATGACTTAGAAGTTATCTC

LeuGluThrProGlnLeuTyrSerGluGluGluLeuLeuAspThrAsnGluSerValGlyGluAsnLeuGlnLeuThrIleThr
TTTAGAAACACCACAATTATATTCAGAAGAAGAGCTTTTAGACACAAACGAAAGTGTGGGCGAAAATTTGCAACTTACTATTACT

AsnSerGluGlyGluValSerIleLeuAspLeuThr
AACTCAGAGGGTGAGGTTTCTATACTAGATTTAAC

GlnSerArgValArgProProPheGlyThrGluAspThrSerLeuHisValTyrTyrProAsnSerSerLysGlyThrProIle
ACAAAGCAGAGTCAGGCCACCTTTTGGCACTGAAGATACTAGCTTGCATGTATATTACCCAAATTCTTCTAAAGGGACTCCAATA

IleAsnProGluGluSerPheThrProLeuValIle
ATTAATCCTGAAGAATCATTTACACCTTTGGTTAT

IleAlaLeuAsnAsnSerThrGlyAspPheGluLeuHisProSerLeuArgLysArgArgLysArgAlaTyrVal***
TATAGCTCTTAACAACTCAACAGGGGATTTTGAGTTACATCCTAGTCTTAGAAAGCGTCGTAAAGAGCTTATGTATAA

Fin L2

début L1 → MetTyrAsnValPheGlnMetAlaValTrpLeuProAlaGlnAsnLysPhe
ATGTATAATGTTTTTCAGATGGCTGTCTGGTTACCAGCGCAGAATAAGT

0 092 456

# FIG.1b.

0 092 456

IleAlaLeuAsnAsnSerThrGlyAspPheGluLeuHisProSerLeuArgLysArgArgLysArgAlaTyrVal<sup>***</sup> Fin L2
TATAGCTCTTAACAACTCAACAGGGGATTTTGAGTTACATCCTAGTCTTAGAAAGCGTCGTAAAAGAGCTTATGTATAA

début L1 → MetTyrAsnValPheGlnMetAlaValTrpLeuProAlaGlnAsnLysPhe
ATGTATAATGTTTTTCAGATGGCTGTCTGGTTACCAGCGCAGAATAAGT
TyrLeuProProGlnProIleThrArgIleLeuSerThrAspGluTyrValThrArgThrAsnLeuPheTyrHisAlaThrSer
TCTATCTTCCTCCCCAGCCCATCACTAGAATCCTGTCCACTGATGAATATGTAACCAGAACCAATCTCTTCTACCATGCAACATCT
GluArgLeuLeuLeuValGlyHisProLeuPheGlu
GAACGTCTACTGCTGGTCGGACATCCTTTGTTTG

IleSerSerAsnGlnThrValThrIleProLysValSerProAsnAlaPheArgValPheArgValArgPheAlaAspProAsn
AGATCTCCAGTAATCAAACTGTAACTATACCAAAAGTGTCACCAAATGCATTTAGAGTTTTTAGGGTGCGTTTTGCTGATCCAAAT
ArgPheAlaPheGlyAspLysAlaIlePheAsnPro
AGATTTGCATTTGGGGATAAGGCAATTTTTAATC

GluThrGluArgLeuValTrpGlyLeuArgGlyIleGluIleGlyArgGlyGlnProLeuGlyIleGlyIleThrGlyHisPro
CAGAAACAGAAAGATTAGTTTGGGGCCTAAGAGGGATAGAGATAGGTAGAGGCCAGCCTTTAGGTATAGGAATAACGGGCCACCCT
LeuLeuAsnLysLeuAspAspAlaGluAsnProThr
CTTTTAAATAAGTTAGATGATGCAGAAAATCCAA

AsnTyrIleAsnThrHisAlaAsnGlyAspSerArgGlnAsnThrAlaPheAspAlaLysGlnThrGlnMetPheLeuValGly
CAAATTATATTAATACTCATGCAAATGGAGATTCTAGACAAAATACTGCTTTTGATGCAAAACAGACACAAATGTTCCTCGTCGGC
CysThrProAlaSerGlyGluHisTrpThrSerSer
TGTACTCCTGCTTCAGGTGAACACTGGACAAGTA

ArgCysProGlyGluGlnValLysLeuGlyAspCysProArgValGlnMetIleGluSerValIleGluAspGlyAspMetMet
GTCGTTGCCCAGGGGAACAAGTGAAACTTGGGGACTGCCCCAGGGTGCAAATGATAGAGTCTGTCATAGAAGATGGTGACATGATG
AspIleGlyPheGlyAlaMetAspPheAlaAlaLeu
GATATTGGTTTTGGGGCTATGGATTTTGCTGCTT

GlnGlnAspLysSerAspValProLeuAspValValGlnAlaThrCysLysTyrProAspTyrIleArgMetAsnHisGluAla
TACAGCAAGACAAGTCTGATGTCCCTTTAGATGTTGTTCAAGCAACATGCAAATATCCTGATTATATCAGAATGAACCATGAAGCC
TyrGlyAsnSerMetPhePhePheAlaArgArgGlu
TATGGCAACTCTATGTTTTTTTTTGCACGTCGCG

# FIG.1c.

GlnMetTyrThrArgHisPhePheThrArgGlyGlySerValGlyAspLysGluAlaValProGlnSerLeuTyrLeuThrAla
AGCAAATGTATACCAGGCACTTTTTTACTCGCGGGGGTTCGGTGGGTGATAAGGAGGCAGTCCCACAAAGCCTGTATTTAACAGCA
AspAlaGluProArgThrThrLeuAlaThrThrAsn
GATGCTGAACCAAGAACAACTTTAGCAACAACAA

TyrValGlyThrProSerGlySerMetValSerSerAspValGlnLeuPheAsnArgSerTyrTrpLeuGlnArgCysGlnGly
ATTATGTAGGCACACCAAGTGGCTCTATGGTTTCATCTGATGTCCAATTGTTTAATAGATCTTACTGGCTTCAGCGATGTCAAGGC
GlnAsnAsnGlyIleCysTrpArgAsnGlnLeuPhe
CAGAATAATGGCATTTGCTGGAGAAACCAGTTAT

IleThrValGlyAspAsnThrArgGlyThrSerLeuSerIleSerMetLysAsnAsnAlaSerThrThrTyrSerAsnAlaAsn
TTATTACAGTTGGAGATAATACCAGAGGAACAAGTTTATCTATCAGTATGAAAAACAATGCAAGTACTACATATTCCAATGCTAAT
PheAsnAspPheLeuArgHisThrGluGluPheAsp
TTTAATGATTTTCTAAGACATACTGAAGAATTTG

LeuSerPheIleValGlnLeuCysLysValLysLeuThrProGluAsnLeuAlaTyrIleHisThrMetAspProAsnIleLeu
ATCTTTCTTTTATAGTTCAGCTTTGTAAAGTAAAGTTAACTCCCGAAAATCTAGCCTACATTCATACAATGGACCCTAATATTTTA
GluAspTrpGlnLeuSerValSerGlnProProThr
GAGGATTGGCAACTATCTGTATCTCAACCACCTA

AsnProLeuGluAspGlnTyrArgPheLeuGlySerSerLeuAlaAlaLysCysProGluGlnAlaProProGluProGlnThr
CCAATCCTCTAGAAGATCAATATAGGTTTTTAGGGTCTTCCTTGGCAGCAAAATGTCCAGAACAGGCGCCTCCTGAGCCCCAGACT
AspProTyrSerGlnTyrLysPheTrpGluValAsp
GATCCTTATAGTCAATATAAATTCTGGGAAGTCG

LeuThrGluArgMetSerGluGlnLeuAspGlnPheProLeuGlyArgLysPheLeuTyrGlnSerGlyMetThrGlnArgThr
ATCTCACAGAAAGGATGTCCGAACAATTAGACCAATTTCCACTAGGAAGGAAATTTCTATATCAAAGTGGCATGACACAACGTACT
AlaThrSerSerThrThrLysArgLysThrValArg
GCTACTAGTTCCACCACAAAGCGCAAAACAGTGC

Fin L1
* * *
ValSerThrSerAlaLysArgArgArgLysAla
GTGTATCTACGTCAGCCAAGCGCAGGCGTAAGGCTTAG.

0 092 456

0 092 456

FIG.2c.

FIG.2a.

FIG.2b.

4

# FIG.3.

HPV1a  Thr  [Glu  [Arg]  Met  [Ser]      Glu  Gln  [Leu  Asp  Gln  Phe  Pro  Leu  Gly  Arg  [Lys
       A C A  G A A  A G G  A T G  T C C      G A A C A A  T T A G A C C A A T T T C C A C T A G G A A G G  A A A

BPV1   A A A  G A A  A A G  C T T  T C T  T T G G A C  T T A G A T C A A T T T C C C T T G G G A A G A  A G A
       Lys  [Glu  [Lys]  Leu  [Ser]  Leu  Asp  Leu  Asp  Gln  Phe  Pro  Leu  Gly  Arg  [Arg


HPV1a  [Phe  Leu]  Tyr  [Gln]      Ser  [Gly]  Met  Thr      Gln  Arg  [Thr]  Ala  Thr  Ser  Ser  Thr
       T T T C T A  T A T  C A A      A G T  G G C  A T G A C A      C A A C G T  A C T  G C T A C T A G T T C C A C C

BPV1   T T T T T A  G C A  C A G  C A A  G G G  G C A G G A T G T T C A      A C T  G T G A G A A A A C G A A G A
       [Phe  Leu]  Ala  [Gln]  Gln  [Gly]  Ala  Gly  Cys  Ser      [Thr]  Val  Arg  Lys  Arg  Arg


HPV1a  Thr  Lys  Arg  [Lys  Thr]  Val  Arg  Val  Ser  Thr  Ser  [Ala  Lys]  [Arg  Arg  Arg  Lys
       A C A A A G C G C  A A A A C A  G T G C G T G T A T C T A C G T C A  G C C A A G  C G C A G G C G T A A G

BPV1   A T T  A G C  C A A  A A A C T  T C C A G T A A G C C T  G C A A A A  A A A A A A A A A A A  T A A  A
       Ile  Ser  Gln  [Lys  Thr]  Ser  Ser  Lys  Pro  [Ala  Lys]  [Lys  Lys  Lys  Lys


HPV1a      Ala
       G C T  T A G  T A T A T A T T A T A T A T A A C T A T A T T T A T T A

BPV1   A G C T A A G T T T C T A T A A A T G T T C T G T A A A T G T A A A A